# FASCICULE DE BREVET EUROPEEN

(11) **EP 0 953 353 B1**
(45) Date de publication et mention de la délivrance du brevet: **25.06.2003**
(21) Numéro de dépôt: 99401033.8
(22) Date de dépôt: 28.04.1999
(51) Int. Cl.: A61K 35/78, A61K 7/48, A61P 17/00

(54) **Utilisation d'oligomères procyanidoliques (OPC) ou d'anthocyanosides à titre d'agents anti-uréase**
Verwendung von procyanidolischen Oligomeren oder Anthocyanosiden als Anti-Urease Wirkstoffe
Use of procyanidolic oligomers or anthocyanosides as anti-urease agents

(30) Priorité: 29.04.1998 FR 9805388
(43) Date de publication de la demande: 03.11.1999
(73) Titulaire: Laboratoires Expanscience, 92419 Courbevoie Cedex (FR)
(72) Inventeur: Msika, Philippe, 75018 Paris (FR); Tep Saly, Thida, 94200 Ivry Sur Seine (FR)
(74) Mandataire: Warcoin, Jacques

(56) Documents cités:
- EP-A- 0 301 975
- EP-A- 0 582 147
- EP-A- 0 692 243
- WO-A-95/21018
- FR-A- 2 461 499
- FR-M- 5 097
- J. MASQUELIER: "Oligomères procyanidoliques" PARFUMS, COSM TIQUES ET AR MES, no. 95, octobre 1990 (1990-10) - novembre 1990 (1990-11), pages 89-97, XP000165236

## Description

La présente invention concerne l'utilisation des oligomères procyanidoliques (OPC) et/ou des anthocyanosides pour la fabrication d'une composition dermatologique pour la prévention ou le traitement des èrythèmes fessiers.

La présente invention concerne également l'utilisation des oligomères procyanidoliques (OPC) et/ou des anthocyanosides autre qu'une méthode de traitement thérapeutique pour la fabrication de compositions cosmétiques comme produits d'hygiène corporelle.

Les oligomères procyanidoliques (également appelés pycnogenols) sont des extraits végétaux titrés en proanthocyanidol dont l'utilisation dans le domaine médical, diététique ou cosmétique a déjà été largement exploitée.

Leur nature et leur composition sont définies par leur mode d'extraction, qui permet d'isoler des végétaux considérés une fraction présentant l'activité biologique recherchée. Les proanthocyanidols responsables de l'activité sont des polyphénols appartenant à la série du flavane-3 ol. Ils se présentent dans la plante sous divers degrés de polymérisation dont certains seulement sont doués d'une activité intéressante. Les extraits bruts de proanthocyanidols sont formés de mélanges complexes comprenant des monomères et des- polymères, plus particulièrement des oligomères allant des dimères et le plus généralement jusqu'aux décamères. Les procédés d'extraction visent à privilégier l'obtention des oligomères c'est-à-dire les formes peu polymérisées, les polymères supérieurs ne présentant généralement comme intérêt que leur pouvoir tannant. En pratique, les oligomères d'intérêt réunissent de deux à quatre molécules mères. L'association des monomères se fait le plus généralement par des liaisons interflavaniques carbone - carbone 4 - 6 ou 4 - 8. Ces polymères ont été isolés ou identifiés dans tous les groupes végétaux, gymnospermes et fougères compris. On les trouve par exemple dans le quebracho, la vigne, le pin, le thé, l'arachide, l'aubépine ou le cyprès. La formule ci-dessous donne un exemple de dimère (procyanidol).

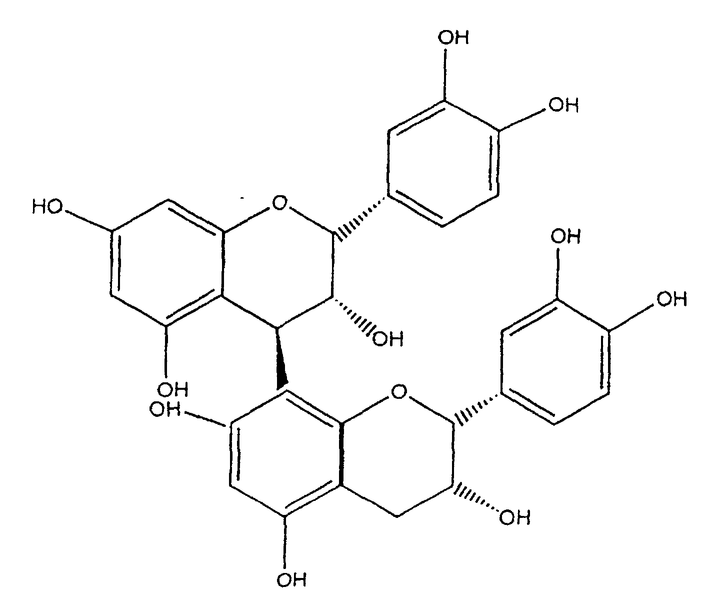

La publication de brevet FR 2 715 582 contient une définition d'OPC utilisables selon l'invention.

D'une manière plus générale, les OPC appartiennent à la famille des tanins définis classiquement comme des composés phénoliques hydrosolubles ayant une masse moléculaire comprise entre 500 et 3000 qui présentent, à côté des réaction classiques des phénols, la propriété de précipiter des alcaloïdes, la gélatine et d'autres protéines (Bate-Smith et Swain, 1962). On distingue maintenant de façon plus précise au sein des tanins, composés polyphénoliques complexes, les proanthocyanidols (tanins condensés pouvant contenir des OPC) et les polyesters de l'acide gallique (tanins hydrolysables).

Les tanins hydrolysables sont des esters d'un sucre (ou d'un polyol apparenté) et d'un nombre variable de molécules d'acide phénolique. Le sucre est très généralement le glucose et l'acide phénolique esc soit l'acide gallique soit l'acide hexahydroxydiphénique (HHDP) et ses dérivés d'oxydation (déhydrohexahydroxydiphénique = DHHDP) dans le cas des tanins classiquement dénommés ellagiques. Depuis 1985, plusieurs représentants d'une nouvelle catégorie de tanins, les tanins complexes ont été isolés. Ces tanins complexes sont des ellagitanins modifiés, résultant de l'addition d'un dérivé phényl-chromatique sur une molécule d'ester HHDP du glucose : flavanol, procyanidol, flavonol. Tanins galliques et ellagiques (simples ou complexes) sont caractéristiques des angiospermes dicotylédones.

En fait, les tanins hydrolysables et les tanins condensés peuvent être distingués sur la base de leur comportement en milieu acide. Les premiers sont hydrolysés, libérant de l'acide gallique ou de l'acide hexahydroxydiphénique (HHDP) qui se lactonise rapidement en acide ellagique. Les seconds conduisent en milieu acide et à chaud à des anthocyanidols par rupture de la liaison interflavannique et oxydation à l'air du flaven-3-ol-3.

La publication du brevet européen EP-A-0 582 147 décrit une composition cosmétique ou dermatologique - anti-uréase en présicant que l'agent anti-uréase est choisi parmi les tanins condensés de caroube et les dérivés de l'acide chlorogénique.

Il est indiqué dans ce document que les tanins condensé de caroube sont constitués d'esters d'acide gallique ou de formes plus complexes de l'acide gallique faisant intervenir des liaisons depsidiques (liaisons entre deux galloyles) et de glucose. Cette publication de brevet décrit également l'utilisation, à titre d'agent anti-uréase, d'un extrait d'Hamamélis qui incorpore des tanins condensés d'Hamamélis constitués d'esters d'acide gallique ou de-formes plus complexes de l'acide gallique et d'hamamélose. Ainsi, bien que ce document qualifie les tanins utilisés en tant qu'agent anti-uréase de tanins condensés, il est clair que la définition qui en est donnée correspond aux tanins hydrolysables tels que définis précédemment.

Ainsi, on doit considérer que le document EP-A-0 582 147 décrit en fait l'utilisation des tanins hydrolysables à titre d'agent anti-uréase.

Par ailleurs, le brevet FR 94 01146 indique que les oligomères procyanidoliques ou OPC peuvent prévenir l'érythème et la couperose. De telles applications des OPC seraient dues à leurs propriétés anti-inflammatoires connues.

Les anthocyanosides constituent une autre famille de produits contenus dans des extraits végétaux et connus pour précipiter les structures protéiques. Il s'agit de pigments hydrosolubles responsables de la coloration rouge, rose, mauve, pourpre, bleue ou violette de la plupart des fleurs et des fruits. Ces pigments existent sous forme d'hétérosides (anthocyanosides) et leurs génines (anthocyanidols) sont des dérivés du cation 2-phénylbenzopyrylium, plus communément appelé cation flavylium, ce qui souligne l'appartenance de ces molécules au vaste groupe des flavonoïdes au sens large. On trouve une définition de ces molécules dans l'ouvrage de J. Bruneton- Pharmacognosie- Phytochimie- plantes médicinales 2è édition- Lavoisier 1993 page 303-311. Parmi les principales plantes à anthocyanosides, on peut citer la myrtille, la cassis et la vigne rouge.

Les inventeurs ont maintenant découvert que les tanins condensés, et d'une manière générale tout extrait végétal contenant des oligomères procyanidoliques (OPC) ainsi que les anthocyanosides avaient une activité anti-uréase, ce qui ouvre la porte à de nouvelles applications de ces produits dans le domaine notamment cosmétique et dermatologique, en particulier pour la prévention et le traitement de l'erythème fessier.

L'invention a donc tout d'abord pour objet l'utilisation d'oligomères procyanidoliques (OPC) et/ou d'anthocyanosides ayant un effet anti-uréase pour la fabrication d'une composition dermatologique pour le traitement ou la prévention des érythèmes fessiers.

Chez les bébés, l'urine n'est qu'un irritant mineur de la peau. Des études réalisées chez des enfants nourris au sein ont montré que l'irritation est essentiellement dûe aux lipases, protéases et uréases contenus dans les selles, celles-ci étant moins actives chez les enfants nourris au sein que celles qui sont retrouvées chez les enfants nourris au biberon.

En effet, le pH des selles de ces bébés est plus acide, grâce à une microflore gastrique plus productive en acide métabolique. Un pH plus élevé, retrouvé dans les selles des bébés nourris au biberon, active les enzymes fécales qui sont dès lors plus agressives pour la peau. Il a été montré que l'irritation de la peau est bien dûe aux enzymes fécales en chauffant les selles : celles-ci sont alors non irritantes sur la peau de l'animal ; le caractère irritant est restauré en additionnant de la trypsine et de la lipase pancréatique à ces selles.

De plus, les lipases fécales facilitent l'attaque protéolytique de la peau et augmentent sa perméabilité aux sels biliaires, qui se sont pourtant révélés non irritants par eux-mêmes dans des études réalisés chez l'animal.

L'augmentation du pH est donc déterminante dans la physiopathologie de l'érythème fessier. C'est la combinaison selles-urines qui apparaît comme étant la plus dommageable. En effet, l'urée contenue dans les urines est dégradée en ammoniaque par l'uréase fécale. L'ammoniaque formé entraîne une alcalinisation du pH, qui ainsi, potentialise l'action des enzymes fécales.

Ainsi, la formation d'ammoniac lors de la dégradation de l'urée par l'uréase joue un rôle important dans la transpiration et l'érythème fessier. Dans le cas particulier de l'érythème fessier, la formation d'ammoniac n'est pas la cause directe de l'érythème mais entraîne une augmentation de pH favorisant l'augmentation de l'activité des lipases et protéases fécales entraînant ainsi une irritation au niveau cutané. C'est pourquoi on peut envisager notamment d'utiliser cette nouvelle propriété anti-uréase des OPC pour prévenir ou traiter la transpiration ou les érythèmes fessiers.

L'activité anti-uréase peut également être mise en oeuvre dans le domaine de l'hygiène intime.

L'invention a également pour objet l'utilisation des OPC et/ou des anthocyanosides, autre qu'une méthode de traitement thérapeutique, pour la fabrication de compositions cosmétiques comme produits d'hygiène corporelle choisis parmi les produits anti-transpirants, les déodorants sous forme de spray ou gel, les shampoings et les savons.

Les anthocyanosides sont de préférence choisis parmi ceux contenus dans la myrtille, le cassis et la vigne rouge, plus particulièrement la myrtille.

Dans le cadre de la présente invention, les OPC peuvent être présents sous forme d'un ou plusieurs extraits végétaux contenant des OPC ou sous une forme purifiée à des degrés divers, à partir d'un ou plusieurs extraits végétaux. Parmi les familles des OPC, on citera plus particulièrement les OPC de Quebracho ou encore les OPC de pépins de raisins ou les OPC de Ratanhia. D'une manière générale, tous les OPC, quelle que soit leur origine végétale, peuvent être utilisés. On peut citer également les OPC du ratanhia (qui contient de 10 à 15 % de tanins condensés), de l'aubépine, du cyprès, du pin maritime, des plantes de la famille des Rosaceae (parties aériennes de l'alchemille vulgaire ; rhizomes de la benoîte, racine et rhizomes du fraisier, organes souterrains de la potentille...).

Dans le cadre de la présente invention, les OPC sont de préférence stabilisés et/ou protégés en vue notamment de faciliter leur administration par voie topique par tout moyen tel que, encapsulation, notamment par des liposomes, des glycosphères, etc...

Selon l'invention, les quantités efficaces d'agent anti-uréase sont celles qui procurent une inhibition de 10 % à 100 % de l'activité de l'uréase mesurée par la méthode indiquée dans l'Exemple 1. Des quantités efficaces sont également celles qui permettent d'obtenir une stabilisation du pH à des valeurs acides ou neutres, ou encore qui permettent de retarder l'apparition d'un pH basique.

Selon un mode de réalisation préféré, la composition utilisée selon l'invention contient de environ 0,005 % à environ 10.% en poids d'OPC et/ou d'anthocyanoside. Plus particulièrement, lorsqu'il s'agit d'OPC, la teneur sera de environ 0,005 % à environ 1 % en poids d'OPC (reportée en poids d'OPC pur), et lorsqu'il s'agit d'anthocyanosides, de environ 0,1 % à environ 10 % en poids.

Au moins un excipient cosmétologiquement ou dermatologiquement acceptable peut être en outre présent dans la composition cosmétique ou dermatologique utilisée selon la présente invention. Une telle composition peut contenir en outre divers additifs tels que des agent colorants, des parfums ou des conservateurs.

La composition cosmétique ou dermatologique selon l'invention peut se présenter sous toutes les formes généralement utilisées dans ce domaine, plus particulièrement de manière à pouvoir être administrée par voie locale : solution, émulsion, crème, pommade, poudre, lait, lotion ou gel conditionné selon le cas en pots ou en tubes, en flacons de verre ou de plastique et/ou éventuellement en flacons doseurs ou encore en ampoules. Les formes cosmétiques sont préparées selon les méthodes usuelles. Pour chaque forme on a recours à des excipients appropriés qui doivent avoir toutes les qualités habituellement requises. En particulier, dans le cas d'une administration locale, il doit être doué d'une grande affinité pour la peau, être parfaitement bien toléré, stable, présenter une consistance adéquate permettant une utilisation facile et agréable.

Les compositions utilisées selon l'invention peuvent également être formulées comme produits d'hygiène corporelle, notamment d'hygiène intime. On peut citer de façon non limitative les produits anti-transpirants, dédorants sous forme de spray ou gel, les shampoings, notamment antipelliculaires, les savons, les produits d'hygiène dentaire etc..

Enfin, selon un mode de réalisation préféré, la composition se présente sous forme d'une crème constituée d'une émulsion eau-dans-l'huile ou huile-dans-l'eau, d'un gel ou d'une pâte à l'eau.

En dermatologie, les applications essentiellement visées sont la prévention et le traitement des érythèmes fessiers. L'invention a donc plus précisément pour objet, l'utilisation d'oligomères procyanidoliques (OPC) et/ou d'anthrocyanosides ayant un effet anti-uréase pour la fabrication d'une composition dermatologique pour le traitement ou la prévention des érythèmes fessiers pour la prévention ou le traitement de l'érythème fessier.

Les exemples suivants illustrent l'invention sans toutefois la limiter et mettent en évidence l'activité anti-uréase de compositions contenant des OPC et/ou des anthocyanosides selon l'invention.

### EXEMPLE 1 : Mesure de l'activité anti-uréase du principe actif seul :

On détermine l'activité anti-uréase à différentes concentrations de principe actif.

La méthode d'analyse de l'activité anti-uréase est basée sur le principe du dosage de l'ammoniac décrit dans la publication du brevet EP-A-582147.

### Méthode

Chaque échantillon contient :
. 1 ml d'une solution d'urée,
. 0,68 ml d'une solution d'uréase,
. le volume de composition testée, correspondant au pourcentage utilisé dans le produit fini. Le volume final est ajusté à 22 ml à l'aide d'un tampon citrate pH 6.

### Réactifs

. solution d'urée : 20 g d'urée (Fluka®) dans 100 ml de tampon citrate à pH 6
. solution d'uréase : 9 mg d'uréase à 100 UI/mg dans 100 ml de tampon citrate à pH 6
. tampon citrate à pH 6 :
   - 47,5 ml d'une solution A constituée de 21,01 g d'acide citrique monohydrate 0,1 M dans 1000 ml d'H₂O ;
   - 202,5 ml d'une solution B constituée de 29,41 g de citrate trisodique 2H20, 0,1 M dans 1000 ml d'H₂O

### Conditions opératoires

Chaque échantillon testé contient 200 g d'urée et 6,12 UI d'uréase.

L'activité anti-uréase est alors mise en évidence in vitro par pHmétrie, en fonction du temps, les échantillons étant incubés à 37°C.

L'urée contenue dans un échantillon de substrat contenant un mélange d'une composition d'urée et d'uréase, est dégradée par l'uréase à 37°C pendant une durée d'incubation imposée de 15 minutes, la dégradation de l'urée par l'uréase conduisant à une augmentation du pH dû à la libération d'ammoniaque.

### EXEMPLE 2 : Activité anti-uréase d'une émulsion huile-dans-l'eau.

On réalise un test in vitro de l'activité anti-uréase d'une crème cosmétique protectrice.

Pour chaque inhibiteur anti-uréase testé, on réalise 3 essais en parallèle :
. Crème+ (1) 1 ml d'urée
. Crème+ (2) 1 ml d'urée + 0,68 ml d'uréase
. Crème+ (3) 1 ml d'urée + inhibiteur au % testé+0,68 ml d'uréase

Les mélanges (1), (2) et (3) sont ajoutés à la crème finie, à 30°C, en même temps, afin que les réactifs se retrouvent bien ensemble dans les mêmes globules. On ajoute du tampon citrate à pH 6 (qs 40 ml).

L'hypothèse de départ est qu'un bébé de poids moyen de 6kg est langé toutes les 4 heures, au cours desquelles il produit en moyenne 40 ml d'urine correspondant à 300 mg d'urée et 30 g de feces contenant une quantité d'uréase variant entre 3 et 30 UI. La quantité de crème à appliquer est évaluée à environ 10 g.

La crème est une émulsion huile-dans-l'eau dont la composition de base, hors principe actif, est indiquée ci-après (la nomenclature utilisée pour les ingrédients est celle de la "Cosmetic, Toiletery and Fragrance Association Inc.", Washington D.C., mise à part la traduction en français)

| | % |
|---|---|
| stéarate de glycérol | 10 |
| octanoate de cétéaryl | 3 |
| huile de palme hydrogénée | 3 |
| huile de tournesol | 4 |
| huile de germes de blé | 0,3 |
| glycérine | 5 |
| eau | qs 100 |
| cyclométhicone | 3 |
| acétate de DL-tocophérol | 0,2 |
| conservateur | 0,2 |

On ajoute à la crème ainsi préparée de l'urée (mélange (1)), un mélange urée-uréase (mélange (2)) ou un mélange urée-uréase principe actif (mélange (3)) à 30°C.

Le tout est placé au bain marie à 37°C sous agitation et le pH est relevé toutes les heures directement dans la crème.

On détermine l'activité de la crème avec le principe actif par comparaison ou avec la crème dépourvue de principe actif.

### EXEMPLE 3 : Activité anti-uréase d'une émulsion eau dans l'huile

L'émulsion eau-dans-huile présente la composition suivante :

| PHASE GRASSE | % |
|---|---|
| beurre de karité | 1 |
| cire d'abeille | 2 |
| Arlacel 1690® (surfactants commercialisés par ICI) | 4 |
| Controx KS (antioxydant) | 0,15 |
| octyl palmitate | 7 |
| huile de vaseline fluide | 10 |
| bentone MIO (épaississant) | 1 |
| oxyde de zinc | 25 |

| PHASE AQUEUSE | |
|---|---|
| eau | qs 100 |
| glycérine | 5 |
| sulfate de magnésium | 0,7 |
| sulfate de zinc | 0,2 |
| sulfate de cuivre | 0,5 |
| OPC Quebreacho encapsulés | 1 |
| Extrait glycolique de Ratanhia | 1 |

Pour préparer la crème on chauffe séparément les composants de la phase aqueuse et de la phase grasse à 70-75°C, l'oxyde de zinc et l'agent épaississant étant incorporés à celle-ci sous forte agitation.

La phase aqueuse est alors ajoutée à la phase grasse, sous forte agitation, maintenue pendant environ 5 minutes. On vérifie l'aspect de l'émulsion au microscope : les globules doivent être de taille homogène.

Le mélange est mis à refroidir et à 40-45°C, on incorpore sous pales le sulfate de cuivre solubilité dans un peu d'eau, les OPC de Quebracho encapsulés et l'extrait glycolique de ratanhia.

La crème obtenue est de couleur rose pâle, un peu brillante et présente une texture fine, facile à appliquer, qui laisse une marque blanche sur la peau.

Mesurée au viscosimètre de BROOKFIELD, fuseau F, vitesse 3, la viscosité est d'environ 220000 centipoises.

Après une période de 40 jours à 50°C, 40°C, 4°C et à température ambiante, la formule est stable, de viscosité régulière et ne présente pas de changement de couleur.

Pour mesurer l'activité anti-uréase, un des trois mélanges (1), (2) ou (3) est ajouté à la crème à 30°C. Le tout est placé au bain-marie à 37°C pendant 1 heure. La phase aqueuse est alors recueillie en cassant l'émulsion avec 2g de chlorure de potassium et 40 ml d'hexane au moyen d'une ampoule à décanter. On relève alors le pH dans cette phase aqueuse toutes les heures. Le chlorure de potassium permet de casser l'émulsion proprement dite et l'hexane solubilise la phase grasse, sans entrainer les extraits glycoliques végétaux qui ont été sélectionnés comme actifs anti-uréase.

Comme à l'exemple 2, on ajoute à la crème ainsi préparée, de l'urée, un mélange urée-uréase ou un mélange urée-principe actif-uréase.

### EXEMPLE 4 :Activité sur une pâte à l'eau

On prépare une pâte à l'eau ayant la composition suivante :

| | % |
|---|---|
| oxyde de Zinc | 20,0 |
| talc | 10,0 |
| carbonate de calcium | 10,0 |
| eau purifiée | qsp 10 |
| glycérine | 30,0 |
| hydroxyethyl cellulose | 0,2 |
| cuivre pidolate | 0,5 |
| zinc pidolate | 0,5 |
| OPC de Quebracho encapsulées | 1,0 |
| extrait glycolique de ratanhia | 1,0 |
| hydroxypropyl guar | 0,1 |

### EXEMPLE 5: Résultats

Les principes actifs testés sont les suivants :
. Extrait sec de myrtille (Indena Della Beffa) (Myrtille, Tableaux 7 et 11)
. Extrait glycolique de ratanhia (Indena Della Beffa) (Ratanhia, Tableaux 1, 6 à 8)
. OPC de Quebracho encapsulé (Radicalys®, Greentech, Tableaux 2 et 7. Dans les émulsions, on utilise le produit Radicalys® encapsulé dans des glycosphères et dans la pâte à l'eau, on utilise le produit Radicalys encapsulé dans des liposomes.
. OPC de pépins de raisins (ADF Chimie) (OPC, Tableau 3)

Les tableaux 1 à 8 ci-après indiquent l'évolution du pH en fonction du temps, qui constitue un bon indicateur de l'activité anti-uréase des compositions testées. Le témoin est un mélange d'urée-uréase en l'absence d'agent anti-uréase.

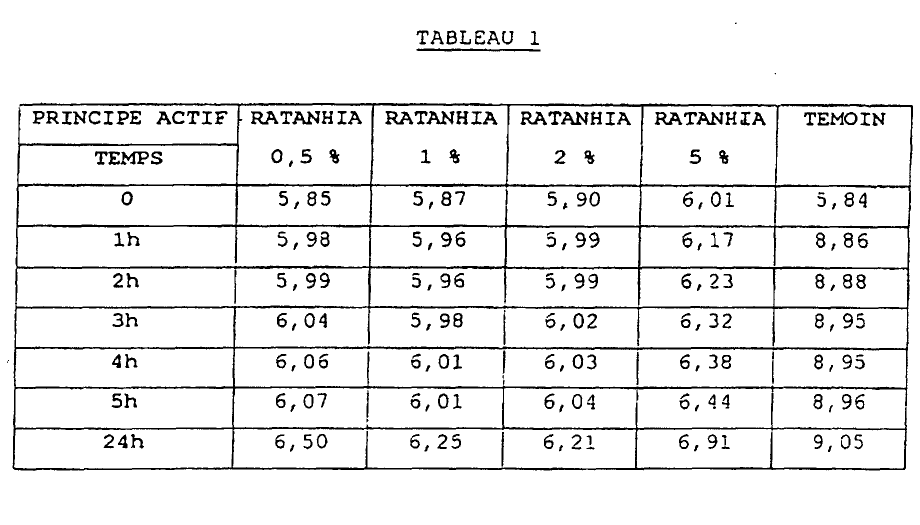

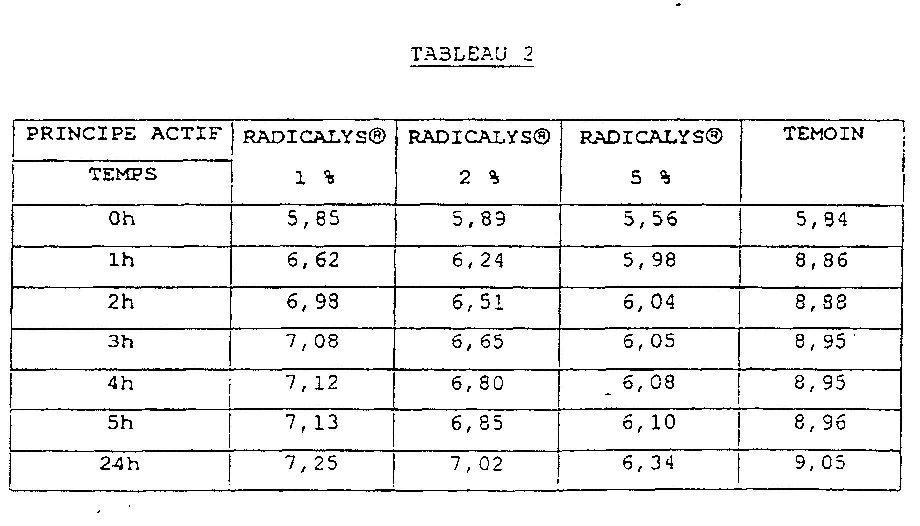

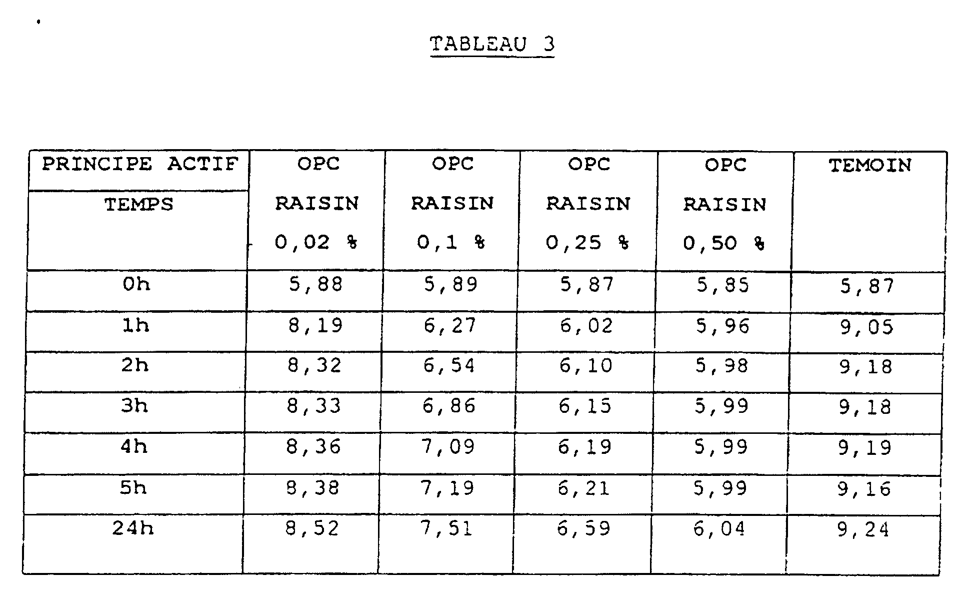

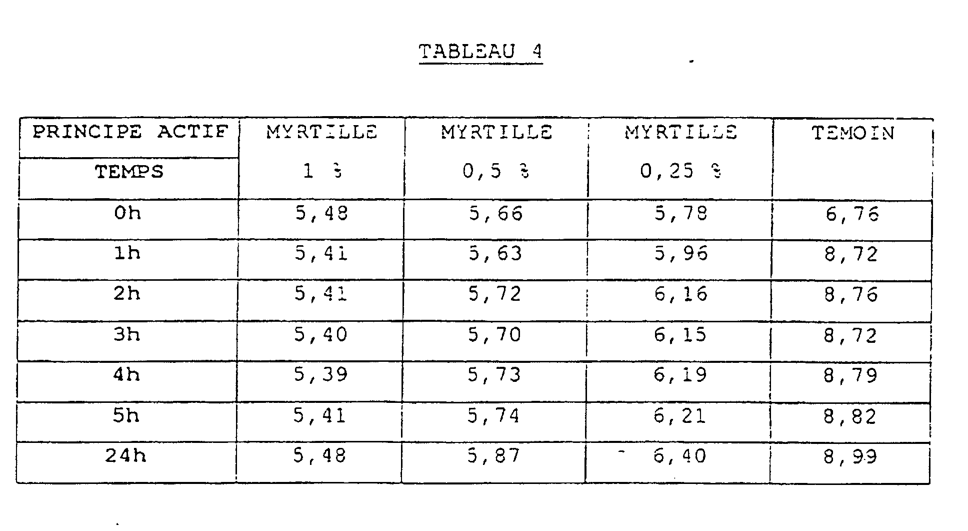

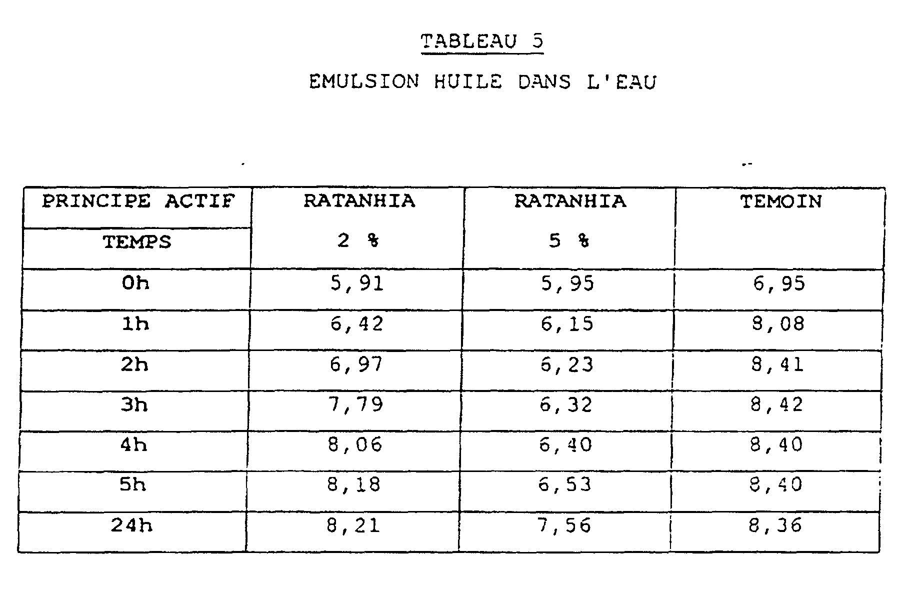

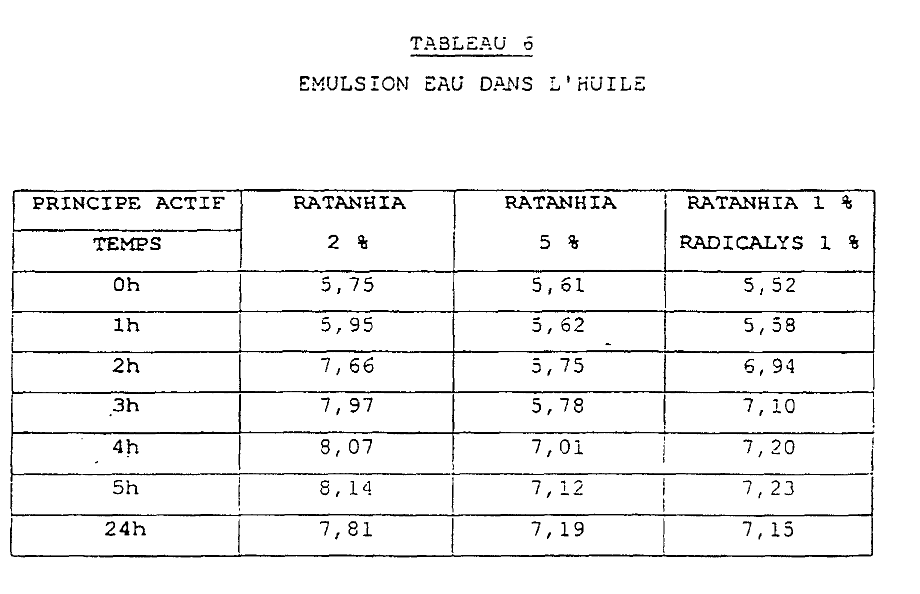

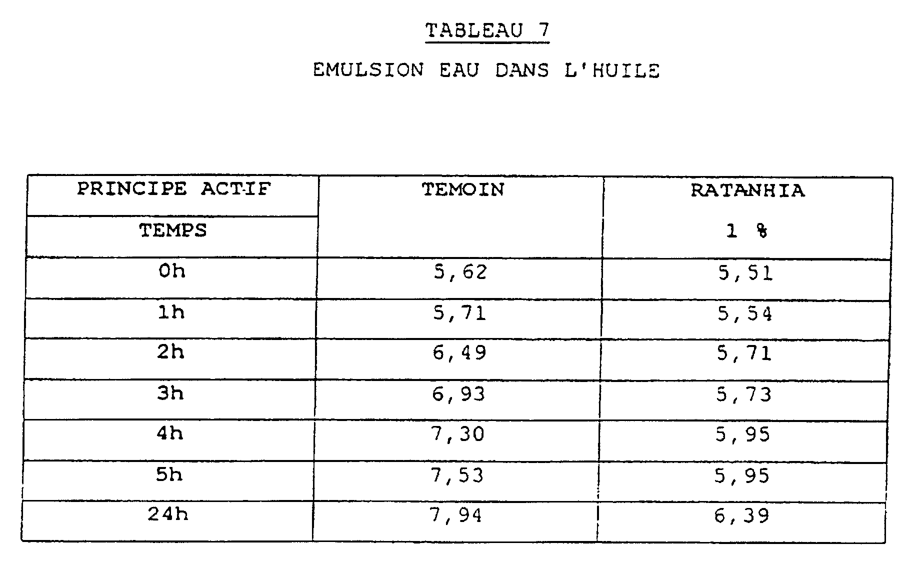

On observe, en se reportant aux tableaux que l'extrait glycolique de ratanhia à 1% présente une bonne activité anti-uréase puisque le pH varie de 5.85 à 6.01 en 4 heures.

Les OPC de Québracho sous forme encapsulée (Radicalys®) sont également actifs à 0,5 % et 1%. L'activité des OPC libres (non protégés) correspondant à 1% de Radicalys® (soit 0,02 % d'OPC purs) est démontrée par une élévation du pH de 5.88 à 8.52 en 4 heures, ces 4 heures étant la durée théorique séparant deux charges.

Dans l'émulsion eau dans huile de la crème protectrice, 1% d'extrait glycolique de ratanhia combiné à 1% de RAdicalys® limite l'augmentation de pH de 5.52 à 7.2 au bout de 4heures alors que 5% d'extrait glycolique de ratanhia sont nécessaires dans la crème pour obtenir un pH de 7.01 au bout du même temps.

Ainsi, les résultats présentés ci-dessus démontrent que l'extrait glycolique de ratanhia ainsi que les OPC encapsulés présentent une bonne activité anti-uréase en milieu liquide (tableaux 1 à 4) ainsi que dans des émulsions huile dans l'eau et eau dans l'huile (tableaux 5 à 7).

## Revendications

1. Utilisation d'oligomères procyanidoliques (OPC) et/ou d'anthocyanosides ayant un effet anti-uréase pour la fabrication d'une composition dermatologique pour le traitement ou la prévention des érythèmes fessiers.

2. Utilisation d'oligomères procyanidoliques (OPC) et/ou d'anthocyanosides autre qu'une méthode de traitement thérapeutique pour la fabrication de compositions cosmétiques comme produits d'hygiène corporelle choisis parmi les produits anti-transpirants, les déodorants sous forme de spray ou gel, les shampoings et les savons.

3. Utilisation selon la revendication 1 ou 2, **caractérisée en ce que** lesdits anthocyanosides sont choisis parmi ceux contenus dans la myrtille.

4. Utilisation selon l'une quelconque des revendications 1 à 3, **caractérisée en ce que** lesdits OPC sont choisis parmi les OPC de pépins de raisin, les OPC de Québracho, et les OPC de Ratanhia.

5. Utilisation selon l'une quelconque des revendications 1 à 4, **caractérisée en ce que** lesdits OPC sont stabilisés et/ou protégés.

6. Utilisation selon la revendication 5, **caractérisée en ce que** lesdits OPC sont stabilisés par encapsulation.

7. Utilisation selon l'une quelconque des revendications 2 à 6, **caractérisée en ce que** la composition cosmétique ou la composition dermatologique contient de 0,005 % à 10 % en poids d'OPC et/ou d'anthocyanoside.

8. Utilisation selon la revendication 7, **caractérisée en ce que** la composition cosmétique ou la composition dermatologique contient de 0,005 % à 1 % en poids d'OPC.

9. Utilisation selon la revendication 7 ou 8, **caractérisée en ce que** la composition cosmétique ou la composition dermatologique contient de 0,1 % à 10 % en poids d'anthocyanosides.

10. Utilisation selon l'une quelconque des revendications 1 à 9, **caractérisée en ce que** la composition cosmétique ou la composition dermatologique contient en outre un excipient cosmétologiquement ou dermatologiquement acceptable.

11. Utilisation selon la revendication 10, **caractérisée en ce que** la composition cosmétique ou la composition dermatologique se présente sous forme d'une solution, émulsion, crème, pommade, poudre, lait, lotion ou gel.

12. Utilisation selon la revendication 11, **caractérisée en ce que** la composition cosmétique ou la composition dermatologique se présente sous forme d'une crème constituée d'une émulsion eau-dans-l'huile ou huile-dans-l'eau, d'un gel ou d'une pâte à l'eau.

13. Utilisation selon l'une quelconque des revendications 10 à 12, **caractérisée en ce que** la composition cosmétique ou la composition dermatologique se présente sous la forme d'un produit d'hygiène corporelle, notamment hygiène intime.

## Patentansprüche

1. Verwendung von procyanidolischen Oligomeren (OPC) und/oder von Anthocyanosiden mit einer Anti-Urease-Wirkung für die Herstellung einer dermatologischen Zusammensetzung für die Behandlung oder Verhinderung von glutäalen Erythemen.

2. Verwendung von procyanidolischen Oligomeren (OPC) und/oder von Anthocyanosiden, eine andere als ein therapeutisches Behandlungsverfahren, für die Herstellung von kosmetischen Zusammensetzungen als Körperpflegeprodukte, ausgewählt unter den Antitranspirant-Produkten, den Deodorants in Form von Spray oder Gel, den Shampoos und den Seifen.

3. Verwendung nach Anspruch 1 oder 2, **dadurch gekennzeichnet, dass** die Anthocyanoside unter jenen, die in der Heidelbeere enthalten sind, ausgewählt sind.

4. Verwendung nach einem der Ansprüche 1 bis 3, **dadurch gekennzeichnet, dass** die OPC unter den OPC von Traubenkernen, den OPC von Quebracho und den OPC von Ratanhia ausgewählt sind.

5. Verwendung nach einem der Ansprüche 1 bis 4, **dadurch gekennzeichnet, dass** die OPC stabilisiert und/oder geschützt sind.

6. Verwendung nach Anspruch 5, **dadurch gekennzeichnet, dass** die OPC durch Verkapselung stabilisiert sind.

7. Verwendung nach einem der Ansprüche 2 bis 6, **dadurch gekennzeichnet, dass** die kosmetische Zusammensetzung oder die dermatologische Zusammensetzung 0,005 Gew.-% bis 10 Gew.-% OPC und/oder Anthocyanosid enthält.

8. Verwendung nach Anspruch 7, **dadurch gekennzeichnet, dass** die kosmetische Zusammensetzung oder die dermatologische Zusammensetzung 0,005 Gew.-% bis 1 Gew.-% OPC enthält.

9. Verwendung nach Anspruch 7 oder 8, **dadurch gekennzeichnet, dass** die kosmetische Zusammensetzung oder die dermatologische Zusammensetzung 0,1 Gew.-% bis 10 Gew.-% Anthocyanoside enthält.

10. Verwendung nach einem der Ansprüche 1 bis 9, **dadurch gekennzeichnet, dass** die kosmetische Zusammensetzung oder die dermatologische Zusammensetzung außerdem einen aus kosmetischer oder dermatologischer Hinsicht annehmbaren Träger enthält.

11. Verwendung nach Anspruch 10, **dadurch gekennzeichnet, dass** die kosmetische Zusammensetzung oder die dermatologische Zusammensetzung in Form einer Lösung, Emulsion, Creme, Pomade, eines Pulvers, einer Milch, Lotion oder eines Gels vorliegt.

12. Verwendung nach Anspruch 11, **dadurch gekennzeichnet, dass** die kosmetische Zusammensetzung oder die dermatologische Zusammensetzung in Form einer Creme, die aus einer Wasser-in-Öl- oder Öl-in-Wasser-Emulsion gebildet wird, eines Gels oder einer wässrigen Paste vorliegt.

13. Verwendung nach einem der Ansprüche 10 bis 12, **dadurch gekennzeichnet, dass** die kosmetische Zusammensetzung oder die dermatologische Zusammensetzung in Form eines Körperpflegeprodukts, insbesondere Intimhygieneprodukts vorliegt.

## Claims

1. Use of procyanidolic oligomers (PCO) and/or of anthocyanosides having an anti-urease effect for the manufacture of a dermatological composition for the treatment or prevention of nappy rash.

2. Use of procyanidolic oligomers (PCO) and/or of anthocyanosides other than a method of therapeutic treatment for the manufacture of cosmetic compositions as body hygiene products chosen from antiperspirant products, deodorants in spray or gel form, shampoos and soaps.

3. Use according to Claim 1 or 2, **characterized in that** the said anthocyanosides are chosen from those contained in bilberry.

4. Use according to any one of Claims 1 to 3, **characterized in that** the said PCOs are chosen from grapeseed PCOs, Quebracho PCOs, and Ratanhia PCOs.

5. Use according to any one of Claims 1 to 4, **characterized in that** the said PCOs are stabilized and/or protected.

6. Use according to Claim 5, **characterized in that** the said PCOs are stabilized by encapsulation.

7. Use according to any one of Claims 2 to 6, **characterized in that** the cosmetic composition or the dermatological composition contains from 0.005% to 10% by weight of PCO and/or of anthocyanoside.

8. Use according to Claim 7, **characterized in that** the cosmetic composition or the dermatological composition contains from 0.005% to 1% by weight of PCO.

9. Use according to Claim 7 or 8, **characterized in that** the cosmetic composition or the dermatological composition contains from 0.1% to 10% by weight of anthocyanosides.

10. Use according to any one of Claims 1 to 9, **characterized in that** the cosmetic composition or the dermatological composition contains, in addition, a cosmetologically or dermatologically acceptable excipient.

11. Use according to Claim 10, **characterized in that** the cosmetic composition or the dermatological composition is provided in the form of a solution, emulsion, cream, ointment, powder, milk, lotion or gel.

12. Use according to Claim 11, **characterized in that** the cosmetic composition or the dermatological composition is provided in the form of a cream consisting of a water-in-oil or oil-in-water emulsion, a gel or a paste containing water.

13. Use according to any one of Claims 10 to 12, **characterized in that** the cosmetic composition or the dermatological composition is provided in the form of a body hygiene, in particular intimate hygiene, product.
